# EUROPÄISCHE PATENTSCHRIFT

(11) **EP 3 143 682 B1**
(45) Veröffentlichungstag und Bekanntmachung des Hinweises auf die Patenterteilung: **31.01.2018**
(21) Anmeldenummer: 15728385.4
(22) Anmeldetag: 13.05.2015
(51) Int. Cl.: H02K 49/10

(54) **MAGNETKUPPLUNG**
MAGNETIC COUPLING
EMBRAYAGE ÉLECTROMAGNÉTIQUE

(30) Priorität: 15.05.2014 AT 503432014
(43) Veröffentlichungstag der Anmeldung: 22.03.2017
(73) Patentinhaber: Technische Universität Wien, 1040 Wien (AT)
(72) Erfinder: JANECZEK, Christoph, A-2603 Felixdorf (AT); HINTEREGGER, Markus, A-1050 Wien (AT)
(74) Vertreter: Sonn & Partner Patentanwälte
(86) Internationale Anmeldenummer: PCT/AT2015/050122
(87) Internationale Veröffentlichungsnummer: WO 2015/172173

(56) Entgegenhaltungen:
- DE-T5-112008 002 854
- US-A1- 2014 077 646
- ZHENG P ET AL: "Force and torque characteristics for magnetically driven blood pump", JOURNAL OF MAGNETISM AND MAGNETIC MATERIALS, ELSEVIER SCIENCE PUBLISHERS, AMSTERDAM, NL, Bd. 241, Nr. 2-3, 1. März 2002 (2002-03-01), Seiten 292-302, XP004351919, ISSN: 0304-8853, DOI: 10.1016/S0304-8853(01)01372-5
- "MAGNETIC COUPLING DELIVERS INCREASED TORQUE", NTIS TECH NOTES, US DEPARTMENT OF COMMERCE. SPRINGFIELD, VA, US, 1. Oktober 1989 (1989-10-01), Seite 863, XP000074110, ISSN: 0889-8464

## Beschreibung

Die Erfindung betrifft eine Magnetkupplung zur Drehmomentübertragung entlang einer Drehachse, mit zwei relativ zueinander drehbaren Kupplungsteilen, wobei ein antriebsseitiger Kupplungsteil einen antriebsseitigen Dauermagnet aufweist und ein abtriebsseitiger Kupplungsteil einen dem antriebsseitigen Dauermagnet entlang der Drehachse gegenüberliegenden und im Abstand davon angeordneten abtriebsseitigen Dauermagnet aufweist, wobei einer der Kupplungsteile ein zumindest teilweise ferromagnetisches Umleitelement umfasst, welches mit dem Dauermagnet des Kupplungsteils drehfest verbunden ist, wobei ein Teil des Umleitelements radial außerhalb des gegenüberliegenden Dauermagnets angeordnet ist. Insbesondere betrifft die Erfindung eine kompakte Magnetkupplung zwischen getrennten Funktionsbereichen ohne Gehäusedurchführung.

Dieser Aufbau entspricht im Wesentlichen dem allgemein als Stirndrehkupplung bekannten Typ einer Magnetkupplung. Charakteristisch für diesen Kupplungstyp sind die axial benachbarten, häufig spiegelbildlich angeordneten, Dauermagnete der beiden Kupplungsteile. In der Regel sind die beiden Kupplungsteile dabei durch eine ebene Trennfläche, welche senkrecht auf die Drehachse steht, getrennt. Eine bekannte Alternative zu diesem Kupplungstyp ist die Zentraldrehkupplung, welche zwei koaxial ineinander angeordnete hohlzylinderförmige Kupplungsteile aufweist. Der grundsätzliche Aufbau beider Kupplungstypen kann beispielsweise der EP 0 039 777 A2 entnommen werden. Zentraldrehkupplungen haben den Vorteil einer besseren Drehmomentübertragung, sind jedoch bei zunehmender Miniaturisierung aufgrund der notwendigen dünnwandigen hohlzylinderförmigen Kupplungsteile und entsprechend flache Dauermagnete nur schwierig und kostenintensiv herstellbar. Andererseits zeigen Stirndrehkupplungen bei einer Verkleinerung der Baugröße eine überproportionale Verschlechterung der Drehmomentübertragung, d.h. mit geringeren Kupplungsabmessungen sinkt nicht nur das verfügbare magnetisierte Volumen sondern auch der für das übertragene Drehmoment entscheidende Radius.

Ein mögliches Einsatzgebiet der erfindungsgemäßen Magnetkupplung, bei dem geringe Abmessungen der Kupplung besonders wünschenswert sind, ist insbesondere die Verwendung als implantiertes Medizingerät, insbesondere als Blutpumpe, vorzugsweise als Herzblutpumpe bzw. als Herzkatheterpumpe vorgesehen. Eine derartige Blutpumpe ist bereits aus der EP 0 904 117 B1 bekannt, wobei die dort gezeigte Magnetkupplung zwischen dem Antrieb und dem Pumpenläufer in an sich bekannter Weise als Stirndrehkupplung ausgeführt ist.
Die DE 11 2008 002854 T5 zeigt eine Kraftübertragungsvorrichtung zum Übertragen einer rotatorischen Antriebskraft. Gemäß einer der Ausführungsformen umfasst diese Kraftübertragungsvorrichtung eine Rolle mit einem antriebsseitigen Dauermagnet sowie einer gegenüberliegenden Nabe mit einem abtriebseitigen Dauermagnet. Die Dauermagnete sind dabei entlang einer Drehachse um die Antriebswelle einander gegenüberliegend angeordnet. Die Rolle weist einen äußeren Ring auf, welcher die Nabe radial außenseitig umgibt und aus einem magnetischen Material, beispielsweise aus Eisen, besteht.
Gegenüber den bekannten Bauformen ist es Aufgabe der Erfindung, eine Magnetkupplung vorzuschlagen, welche bei vorgegebenen, besonders kompakten Abmessungen einerseits einfacher und kostengünstiger als vergleichbare Zentraldrehkupplungen herstellbar ist und andererseits eine gegenüber herkömmlichen Stirndrehkupplungen gesteigerte Effizienz hinsichtlich des übertragenen Drehmoments aufweist.

Diese Aufgabe wird bei der in Anspruch 1 definierten erfindungsgemässen Magnetkupplung dadurch gelöst, dass das Umleitelement zumindest eine diamagnetische Trennung aufweist, welche das Umleitelement in zumindest zwei ferromagnetische Abschnitte unterteilt. Mit einer solchen Trennung kann ein magnetischer Kurzschluss im Umleitelement vermieden werden. Das Umleitelement kann dabei - vergleichbar dem äußeren Kupplungsteil einer Zentraldrehkupplung - topf- bzw. hohlzylinderförmig ausgebildet sein und den jeweils anderen Kupplungsteil umfangseitig umgeben, d.h. es erstreckt sich vorzugsweise radial außerhalb beider Dauermagnete. Dabei kann das Umleitelement beispielsweise als dünnwandiger Hohlzylinder ausgebildet sein, sodass bei gleichbleibenden Abmessungen das magnetisierte Volumen der Stirndrehkupplung weitestgehend erhalten bleibt und zugleich zwischen dem Umleitelement und dem im Abstand davon gegenüberliegenden Dauermagnet ein einer Zentraldrehkupplung vergleichbar großes übertragbares Drehmoment erzielbar ist. Die Magnetisierungsrichtung der Dauermagnete ist dabei vorzugsweise senkrecht auf die Drehachse ausgerichtet, d.h. die Magnetpole verlaufen in Umfangsrichtung von Süd nach Nord und liegen sich - jedenfalls bei einer zweipoligen Ausführung - bezüglich der Drehachse diametral gegenüber. Durch das Umleitelement werden radial von den Dauermagneten ausgehende magnetische Feldlinien gebündelt und die magnetische Kraft zwischen den Kupplungsteilen wegen des ferromagnetischen Materials des Umleitelements zusätzlich verstärkt. Durch die Verdichtung der magnetischen Feldlinien im ferromagnetischen Material wird die magnetische Kraft zur Übertragung des Drehmoments vergrößert. Aufgrund des im Vergleich zu Zentraldrehkupplungen bei gleichen Kupplungsabmessungen größeren Volumens der Dauermagneten kann vorteilhafter Weise eine geringere axiale Erstreckung und somit geringere radiale Querkräfte auf die Lager der Kupplungsteile erzielt werden. Weitere vorteilhafte Ausgestaltungsformen der Erfindung sind in den abhängigen Ansprüchen 2 - 10 definiert. Eine kompakte Herstellung der Magnetkupplung mit einer zugleich vergleichsweise guten Drehmoment-Übertragungsfähigkeit kann erzielt werden, wenn die beiden Dauermagnete jeweils 2-, 4- oder 6-polige Dauermagnete sind. Um die Übertragungsfähigkeit des Drehmoments der Magnetkupplung zu optimieren, wird insbesondere in Abhängigkeit vom Durchmesser der Kupplung die jeweilige Polanzahl für beide Magnete festgelegt werden. Bei verhältnismäßig großen Magnetkupplungen ist auch eine höhere Polanzahl möglich. Im Falle der 2-poligen Ausgestaltung können die Dauermagnete jeweils zwei halbzylinderförmige Magnetpole aufweisen.

Im Fall eines zweipoligen Dauermagnets kann die Trennung als diamagnetischer Trennstreifen entlang einer den Dauermagnet zentral und quer zur Magnetisierungsrichtung schneidenden Ebene ausgeführt sein, d.h. der Trennstreifen teilt das Umleitelement in zwei Hälften.

Wenn sich das Umleitelement an eine dem gegenüberliegenden Dauermagnet abgewandten Rückseite des drehfest verbundenen Dauermagnets erstreckt, kann die Magnetisierung des Umleitelements und somit das übertragbare Drehmoment zusätzlich gesteigert werden.

Darüber hinaus hat es sich als günstig herausgestellt, wenn das Umleitelement einen hohlzylinderförmigen Mantel aufweist und vorzugsweise mit einem auf im Wesentlichen halber Höhe des Mantels angeordneten Zwischenboden ausgebildet ist. Das Umleitelement weist in diesem Fall einen im Wesentlichen H-förmigen Längsschnitt auf, wobei der Zwischenboden den senkrecht auf die Drehachse liegenden Quersteg bildet, so dass beidseitig des Zwischenbodens topfförmige Ausnehmungen gebildet sind. In einer dieser Ausnehmungen ist ein Dauermagnet aufgenommen und drehfest verbunden.

Eine besonders hohe Konzentration von Magnetfeldlinien im Umleitelement kann erzielt werden, wenn an einer dem gegenüberliegenden Dauermagnet abgewandten Rückseite des drehfest mit dem Umleitelement verbundenen Dauermagnets ein diamagnetisches Abschirmelement angeordnet ist. Dadurch können außerhalb der Kupplungsteile verlaufende Feldlinien vermieden und damit verbundene Verluste reduziert werden.

Weiters hat es sich als günstig herausgestellt, wenn an einer dem gegenüberliegenden Dauermagnet zugewandten Vorderseite des drehfest mit dem Umleitelement verbundenen Dauermagnets, insbesondere in einem um die Drehachse zentrierten Bereich, ein diamagnetisches Abschirmelement angeordnet ist, welches vorzugsweise umfangsseitig bzw. radial außenseitig an das Umleitelement anschließt. Mit einer derartigen Abschirmung kann eine Umlenkung des magnetischen Feldes in radial in größerem Abstand von der Drehachse befindliche Bereiche erzielt werden, sodass das bei gegebener Magnetkraft übertragene Drehmoment erhöht wird.

Für eine Drehmomentübertragung zwischen getrennten Funktionsbereichen, z.B. bei Pumpenanwendungen mit einem im Pumpmedium gelagerten Pumpenläufer, ist es günstig, wenn die beiden Kupplungsteile hermetisch getrennt sind. Eine solche hermetische Trennung kann etwa durch eine hermetische Wand zwischen den beiden Kupplungsteilen erzielt werden, welche Wand weder magnetisch noch elektrisch leitend sein sollte. Diese muss nicht zwingend Teil eines Gehäuses der Kupplung sein, kann aber beispielsweise an ein Gehäuse anschließen. Grundsätzlich kann die vorliegende Magnetkupplung aber auch ohne hermetische Trennung, beispielsweise für Sicherheitskupplungen, d.h. zur Begrenzung des übertragenen Drehmoments, eingesetzt werden.

Im Zusammenhang mit einer hermetischen Trennung der Kupplungsteile ist es vorteilhaft, wenn zur hermetischen Trennung der beiden Kupplungsteile zumindest einer der Kupplungsteile in einem im Wesentlichen nichtmagnetischen und elektrisch nicht leitenden Gehäuse untergebracht ist. Mit einem solchen Gehäuse können Verluste aufgrund einer Ummagnetisierung des Gehäuses bzw. induzierte Wirbelströme im Gehäuse vermieden werden.

Die vorliegende Magnetkupplung kann besonders vorteilhaft in einer Pumpe mit einem Antrieb und einem Pumpenläufer verwendet werden, wobei der Pumpenläufer über die Magnetkupplung mit dem Antrieb verbunden ist. Die so erzielte Pumpe kann besonders kompakt ausgeführt sein, bei zugleich relativ hohem übertragenem Drehmoment mit einer entsprechend vorteilhaften Pumpleistung.

Eine Verwendung, bei der besonders kompakte Abmessungen der Pumpe erforderlich sind, betrifft implantierte Medizingeräte, insbesondere Blutpumpen, vorzugsweise Herzblutpumpen. Zugleich ist eine hermetische Trennung des Antriebs vom Pumpenläufer vorteilhaft, wobei zugleich ein möglichst hohes Drehmoment übertragen werden soll. Diese Anforderungen werden von der hier vorgeschlagenen Magnetkupplung besonders gut erfüllt.

Die Erfindung wird nachfolgend anhand von besonders bevorzugten Ausführungsbeispielen, auf die sie jedoch nicht beschränkt sein soll, und unter Bezugnahme auf die Zeichnungen noch weiter erläutert. In den Zeichnungen zeigen dabei im Einzelnen:
Fig. 1 eine schaubildliche Ansicht einer Magnetkupplung zur Übertragung eines Drehmoments mit einem rückseitig kegelstumpfförmig abgeschrägten Umleitelement;
Fig. 2 einen Längsschnitt durch eine Magnetkupplung gemäß Fig. 1, wobei die beiden Kupplungsteile durch ein Gehäuse getrennt sind;
Fig. 3 eine Seitenansicht auf eine Rückseite des antriebsseitigen Kupplungsteils gemäß Fig. 1 und Fig. 2, jedoch ohne Gehäuse;
Fig. 4 einen Längsschnitt durch eine Variante der Magnetkupplung gemäß Fig. 2 mit einem diamagnetischen Abschirmelement an einer Vorderseite des antriebsseitigen Kupplungsteils;
Fig. 5 eine schaubildliche Ansicht einer Variante einer Magnetkupplung zur Übertragung eines Drehmoments mit einem Umleitelement ohne eine rückseitige kegelstumpfförmige Abschrägung des Umleitelements;
Fig. 6 einen Längsschnitt durch eine Magnetkupplung gemäß Fig. 5, wobei die beiden Kupplungsteile durch ein Gehäuse getrennt sind und mit einem diamagnetischen Abschirmelement an einer Rückseite des antriebsseitigen Kupplungsteils;
Fig. 7 eine Seitenansicht auf eine Rückseite des antriebsseitigen Kupplungsteils gemäß Fig. 5 und Fig. 6 ohne Gehäuse;
Fig. 8 einen Längsschnitt durch eine Magnetkupplung gemäß Fig. 5, wobei das Umleitelement einen Zwischenboden aufweist, welcher an einer Vorderseite des antriebsseitigen Kupplungsteils angeordnet ist und mit einer diamagnetischen Abschirmung an dessen Rückseite;
Fig. 9 eine Seitenansicht auf eine Rückseite des antriebsseitigen Kupplungsteils gemäß Fig. 5 und Fig. 8 ohne Gehäuse;
Fig. 10 einen Längsschnitt durch eine Magnetkupplung gemäß Fig. 8, wobei die diamagnetische Abschirmung durch einen ferromagnetischen Boden ersetzt ist; und
Fig. 11 eine Seitenansicht auf eine Rückseite des antriebsseitigen Kupplungsteils gemäß Fig. 5 und Fig. 10 ohne Gehäuse.

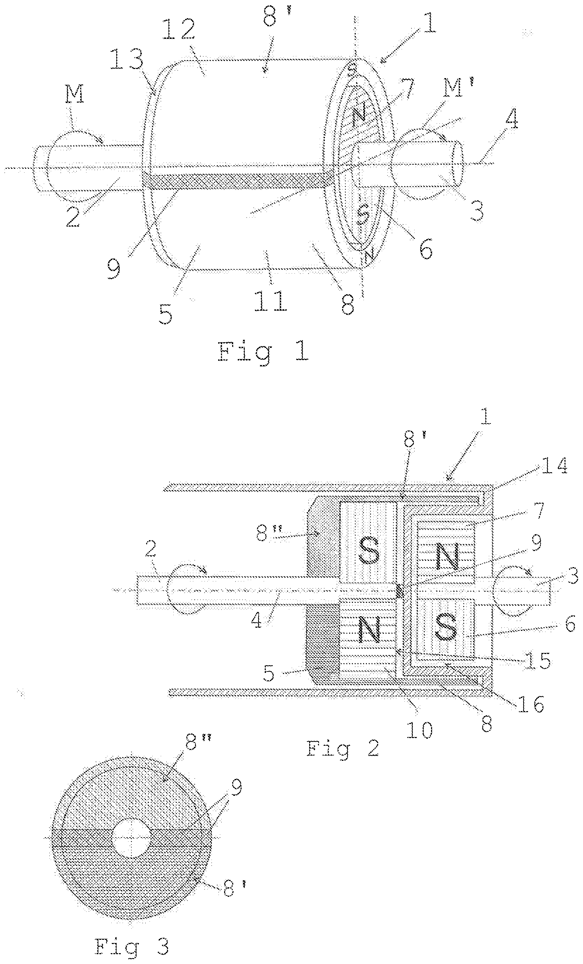

In Fig. 1 ist eine Magnetkupplung 1 dargestellt, welche eine Antriebswelle 2 mit einer Abtriebswelle 3 zur berührungslosen Übertragung eines Drehmoments M, M' verbindet. Die beiden Wellen 2, 3 liegen auf einer gemeinsamen Drehachse 4, sodass ein antriebsseitiger Kupplungsteil 5 relativ zu einem abtriebsseitigen Kupplungsteil 6 drehbar gelagert ist. Der abtriebsseitige Kupplungsteil 6 umfasst einen abtriebsseitigen zweipoligen Dauermagnet 7, welcher drehfest mit der Abtriebswelle 3 verbunden ist, insbesondere auf die Abtriebswelle 3 aufgesteckt ist (vgl. Fig. 2). Der abtriebsseitige Dauermagnet 7 ist umfangseitig von einem im Wesentlichen topfförmigen Umleitelement 8 mit einem hohlzylinderförmigen Mantel 8' und einem den Mantel 8' an einem Ende abschließenden, scheibenförmigen, im Wesentlichen flachen Boden 8" (vgl. Fig. 2) umgeben. Dabei ist zwischen dem abtriebsseitigen Dauermagnet 7 und dem Umleitelement 8 ein Abstand bzw. Spalt vorgesehen, sodass der abtriebsseitige Kupplungsteil 6 berührungslos mit dem antriebsseitigen Kupplungsteil 5 gekoppelt ist. Das Umleitelement 8 besteht größtenteils aus ferromagnetischem Material. Der Mantel 8' des Umleitelements 8 ist lediglich in einem schmalen Winkelbereich durch eine diamagnetische Trennung 9 unterbrochen und die Trennung 9 erstreckt sich außerdem quer über den Boden 8''. Die Trennung 9 teilt das Umleitelement 8 im Wesentlichen in zwei ferromagnetische Hälften bzw. Halbschalen. Eine durch die Trennung 9 verlaufende Schnittebene steht somit senkrecht auf eine Magnetisierungsrichtung des mit dem Umleitelement 8 verbundenen antriebsseitigen zweipoligen Dauermagnets 10 (vgl. Fig. 2). Die ferromagnetischen Abschnitte 11, 12 des Umleitelements 8 sind daher entsprechend dem antriebsseitigen Dauermagnet 10 magnetisiert. Im Fall von mehrpoligen Dauermagneten 7, 10 wären entsprechende zusätzliche Trennungen im Umleitelement 8 erforderlich, um eine optimale Magnetisierung des Umleitelements 8 zu ermöglichen. Mit der antriebsseitig kegelstumpfförmigen Abschrägung 13 des Umleitelements 8 wird ein möglichst homogener Magnetfeldgradient im Umleitelement 8 erzielt, bzw. werden etwaige durch Inhomogenitäten an den Kanten hervorgerufene Magnetisierungsverluste reduziert.

In Fig. 2 ist die in Fig. 1 gezeigte Magnetkupplung 1 im Längsschnitt dargestellt, wobei zusätzlich der antriebsseitige Kupplungsteil 5 durch ein Gehäuse 14 vom abtriebsseitigen Kupplungsteil 6 getrennt ist. Das Gehäuse 14 bildet dabei eine hermetische Trennung zwischen den Funktionsbereichen der beiden Kupplungsteile 5, 6. Das antriebsseitige Kupplungsteil 5 mit dem antriebsseitigen Dauermagnet 10 und dem Umleitelement 8 ist drehbar im Gehäuse 14 aufgenommen, wobei das Gehäuse 14 der topfförmigen Ausnehmung im Umleitelement 8 annähernd bis zu einer Vorderseite 15 des antriebsseitigen Dauermagnets 10 folgt und selbst eine entsprechend kleinere topfförmige Ausnehmung 16 zur Aufnahme des abtriebsseitigen Kupplungsteils 6 bildet. Da der Innendurchmesser des Mantels 8' des Umleitelements 8 naturgemäß größer als der Außendurchmesser des darin drehbar angeordneten gegenüberliegenden Kupplungsteils 6 bzw. dessen Dauermagnets 7 ist und zugleich der Dauermagnet 10 des mit dem Umleitelement 8 verbundenen Kupplungsteils 5 den Mantel 8' vorteilhafter Weise radial vollständig ausfüllt, ist dessen Außendurchmesser im Allgemeinen größer als jener des gegenüberliegenden Dauermagnets 7. Durch den Boden 8" des Umleitelements 8 aus ferromagnetischem Material werden etwaige zur Rückseite des Dauermagnets 10 austretende Feldlinien über das Umleitelement 8 zur Vorderseite umgelenkt und tragen somit zur Drehmomentübertragung bei. Wie weiters in Fig. 2 ersichtlich, sind die beiden Dauermagnete 7, 10 auf die jeweils zugeordneten Wellen 3, 2 aufgesteckt bzw. entlang der Drehachse 4 von den Wellen 3, 2 durchsetzt.

In Fig. 3 ist die Magnetkupplung 1 mit Blick auf den Boden 8" des Umleitelements 8 dargestellt, wobei die quer über den Boden 8" verlaufende diamagnetische Trennung 9, welche den Boden 8" in zwei halbkreisförmige Hälften teilt, ersichtlich ist.

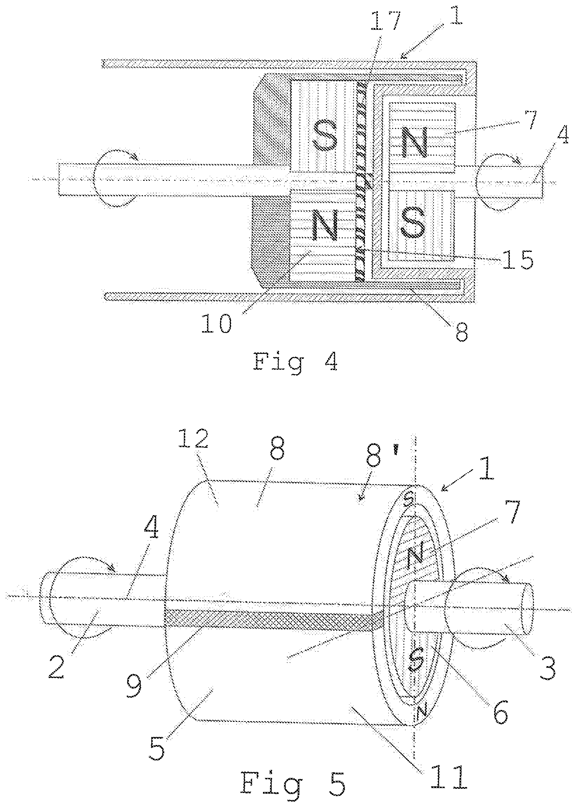

Fig. 4 zeigt eine gegenüber Fig. 2 erweiterte Variante der Magnetkupplung 1, wobei die Vorderseite 15 des mit dem Umleitelement 8 verbundenen Dauermagnets 10 mit einem diamagnetischen Abschirmelement 17 versehen ist. Durch das Abschirmelement 17 wird einem Schluss der magnetischen Feldlinien zwischen den beiden Dauermagneten 7, 10 bei geringen Radien, d.h. in der Nähe der Drehachse 4, entgegengewirkt.

Dadurch wird eine Verschiebung der wirkenden Magnetkraft zu größeren Radien, insbesondere zum Umleitelement 8, und somit eine effizientere Drehmomentübertragung erzielt.

Bei den im Folgenden beschriebenen Varianten der Magnetkupplung 1, welche der in Fig. 5 gezeigten schaubildlichen Ansicht entsprechen, ist im Unterschied zu Fig. 1 konstruktionsbedingt an der Rückseite des Umleitelements 8 keine kegelstumpfförmige Abschrägung vorgesehen. Ansonsten ist der grundsätzliche Aufbau identisch mit der in Fig. 1 gezeigten Magnetkupplung 1.

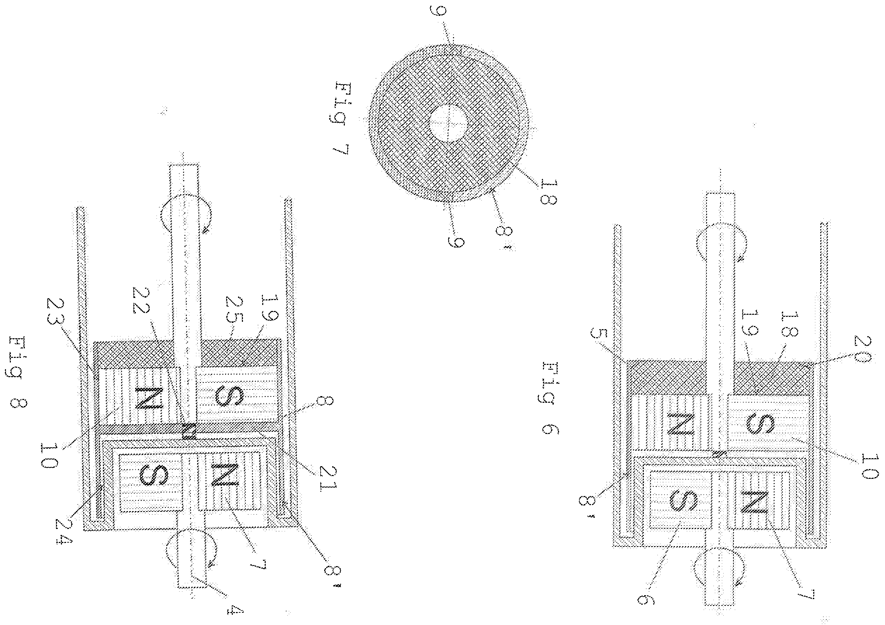

In Fig. 6 und 7 ist eine Variante der Magnetkupplung 1 gezeigt, bei der der ferromagnetische Boden 8" des Umleitelements 8 (vgl. Fig. 2 und 4) durch ein diamagnetisches Abschirmelement 18 an der Rückseite 19 des mit dem Umleitelement 8 verbundenen Dauermagnets 10 ersetzt ist. Durch das Abschirmelement 18 werden rückseitig austretende Feldlinien des Magnetfelds zwischen den Dauermagneten 7, 10 der beiden Kupplungsteile 5, 6 vermieden, sodass an der Außen- und Vorderseite ein entsprechend höheres Magnetfeld erzielt wird. Eine Trennung 9 wie beim ferromagnetischen Boden 8" (vgl. Fig. 3) ist bei dem Abschirmelement 18 nicht erforderlich. Zur Befestigung des Abschirmelements 18 im Umleitelement 8 weist der Mantel 8' des Umleitelements 8 am geschlossenen Ende eine Verjüngung 20 auf, in welche das Abschirmelement 18 eingepasst ist. Der Mantel 8' umgibt demzufolge sowohl den damit drehfest verbundenen Dauermagnet 10 und den gegenüberliegenden Dauermagnet 7 als auch das Abschirmelement 18.

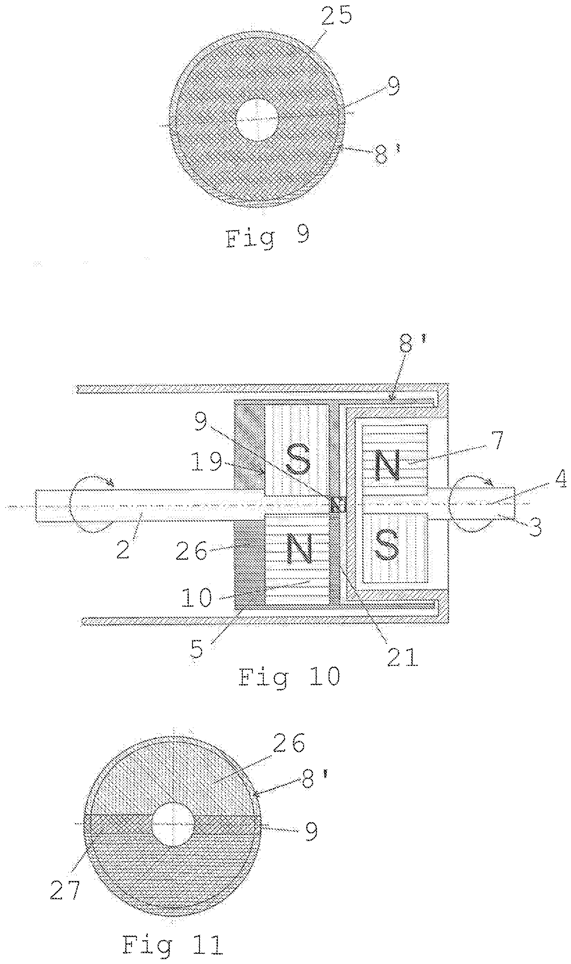

Eine weitere alternative Ausführungsform der Magnetkupplung 1 ist in Fig. 8 und 9 gezeigt, wobei das Umleitelement 8 hier anstelle des Bodens 8" einen Zwischenboden 21 etwa auf halber Höhe des Mantels 8' aufweist. Der Zwischenboden 21 weist - wie der Boden 8" (vgl. Fig. 3) - einen quer durch die Drehachse 4 verlaufenden Trennstreifen 22 auf. Beidseitig des Zwischenbodens 21 bildet das Umleitelement 8 somit topfförmige Ausnehmungen 23, 24 aus, wobei in die antriebsseitige Ausnehmung 23 der antriebsseitige Dauermagnet 10 und im Anschluss daran ein scheibenförmiges diamagnetisches Abschirmelement 25 aufgenommen und drehfest verbunden ist. Die abtriebsseitige Ausnehmung 24 umgibt dementsprechend den abtriebsseitigen Dauermagnet 7. Gegenüber der in Fig. 6 gezeigten Variante der Magnetkupplung 1 bietet die Variante gemäß Fig. 8 eine bessere Formstabilität des Umleitelements 8 und somit eine höhere mechanische Belastbarkeit der Magnetkupplung 1 im Sinne der Fertigung.

Die in Fig. 10 und 11 gezeigte Variante der Magnetkupplung 1 entspricht im Wesentlichen der in Fig. 8 und 9 gezeigten Ausführungsform, wobei lediglich anstelle des diamagnetischen Abschirmelements 25 ein ferromagnetischer Boden 26 vergleichbar dem Boden 8" des Umleitelements 8 gemäß Fig. 2 und 3 in die antriebsseitige Ausnehmung 23 eingesetzt ist. Der eingesetzte Boden 26 weist dabei zweckmäßig ebenfalls einen diamagnetischen Trennstreifen 27 auf, welcher den Boden 26 in zwei halbkreisförmige Hälften teilt und so einen Schluss des magnetischen Kreises zwischen den beiden Polen des Dauermagnets 10 über die Rückseite des Kupplungsteils 5 verhindert.

## Patentansprüche

1. Magnetkupplung (1) zur Drehmomentübertragung entlang einer Drehachse (4), mit zwei relativ zueinander drehbaren Kupplungsteilen (5, 6), wobei ein antriebsseitiger Kupplungsteil (5) einen antriebsseitigen Dauermagnet (10) aufweist und ein abtriebsseitiger Kupplungsteil (6) einen dem antriebsseitigen Dauermagnet (10) entlang der Drehachse (4) gegenüberliegenden und im Abstand davon angeordneten abtriebsseitigen Dauermagnet (7) aufweist, wobei einer der Kupplungsteile (5; 6) ein zumindest teilweise ferromagnetisches Umleitelement (8) umfasst, welches mit dem Dauermagnet (10; 7) des Kupplungsteils (5; 6) drehfest verbunden ist, wobei ein Teil des Umleitelements (8) radial außerhalb des gegenüberliegenden Dauermagnets (7; 10) angeordnet ist, **dadurch gekennzeichnet, dass** das Umleitelement (8) zumindest eine diamagnetische Trennung (9) aufweist, welche das Umleitelement (8) in zumindest zwei ferromagnetische Abschnitte (11, 12) unterteilt.

2. Magnetkupplung (1) nach Anspruch 1, **dadurch gekennzeichnet, dass** die beiden Dauermagnete (10, 7) jeweils 2-, 4-, oder 6-polige Dauermagnete sind.

3. Magnetkupplung (1) nach Anspruch 1 oder 2, **dadurch gekennzeichnet, dass** sich das Umleitelement (8) an eine dem gegenüberliegenden Dauermagnet (7; 10) abgewandten Rückseite (19) des drehfest verbundenen Dauermagnets (10; 7) erstreckt.

4. Magnetkupplung (1) nach einem der Ansprüche 1 bis 3, **dadurch gekennzeichnet, dass** das Umleitelement (8) einen hohlzylinderförmigen Mantel (8') aufweist und vorzugsweise mit einem auf im Wesentlichen halber Höhe des Mantels (8') angeordneten Zwischenboden (21) ausgebildet ist.

5. Magnetkupplung (1) nach einem der Ansprüche 1 bis 4, **dadurch gekennzeichnet, dass** an einer dem gegenüberliegenden Dauermagnet (7) abgewandten Rückseite (19) des drehfest mit dem Umleitelement (8) verbundenen Dauermagnets (10) ein diamagnetisches Abschirmelement (18, 25) angeordnet ist.

6. Magnetkupplung (1) nach einem der Ansprüche 1 bis 5, **dadurch gekennzeichnet, dass** an einer dem gegenüberliegenden Dauermagnet (7) zugewandten Vorderseite (15) des drehfest mit dem Umleitelement (8) verbundenen Dauermagnets (10), insbesondere in einem um die Drehachse (4) zentrierten Bereich, ein diamagnetisches Abschirmelement (17) angeordnet ist, welches vorzugsweise umfangseitig an das Umleitelement (8) anschließt.

7. Magnetkupplung (1) nach einem der Ansprüche 1 bis 6, **dadurch gekennzeichnet, dass** die beiden Kupplungsteilen (5, 6) hermetisch getrennt sind.

8. Magnetkupplung (1) nach Anspruch 7, **dadurch gekennzeichnet, dass** zur hermetischen Trennung der beiden Kupplungsteile zumindest einer der Kupplungsteile (5, 6) in einem im Wesentlichen nichtmagnetischen und nicht elektrisch leitenden Gehäuse (14) untergebracht ist.

9. Pumpe mit einem Antrieb und einem Pumpenläufer, wobei der Pumpenläufer über eine Magnetkupplung (1) nach einem der Ansprüche 1 bis 8 mit dem Antrieb verbunden ist.

10. Verwendung einer Pumpe nach Anspruch 9 als Medizingerät, insbesondere als implantierte Blutpumpe, vorzugsweise als Herzblutpumpe.

## Claims

1. A magnetic coupling (1) for the transmission of torque along a rotary axis (4) with two coupling portions (5, 6) rotatable relative to one another, wherein a drive side coupling portion (5) includes a drive side permanent magnet (10) and an output side coupling portion (6) includes an output side permanent magnet (7) opposed to the drive side permanent magnet (10) along the rotary axis and spaced from it, wherein one of the coupling portions (5; 6) includes an at least partially ferromagnetic diverting element (8), which is rotationally fixedly connected to the permanent magnet (10; 7) of the coupling portion (5; 6), wherein a portion of the diverting element (8) is arranged radially outside the opposed permanent magnet (7; 10), **characterised in that** the diverting element (8) includes at least one diamagnetic separator (9), which divides the diverting element (8) into at least two ferromagnetic sections (11, 12).

2. A magnetic coupling (1) as claimed in Claim 1, **characterised in that** the two permanent magnets (10, 7) are in each case 2-, 4-, or 6-pole permanent magnets.

3. A magnetic coupling (1) as claimed in Claim 1 or 2, **characterised in that** the diverting element (8) extends on a rear surface (19) of the rotationally fixedly connected permanent magnet (10; 7) remote from the opposed permanent magnet (7; 10).

4. A magnetic coupling (1) as claimed in one of Claims 1 to 3, **characterised in that** the diverting element (8) includes a hollow cylindrical shell (8') and is preferably constructed with an intermediate base arranged substantially half way up the height of the shell (8').

5. A magnetic coupling (1) as claimed in one of Claims 1 to 4, **characterised in that** a diamagnetic shielding element (18, 25) is arranged on a rear surface, remote from the opposed permanent magnet (7), of the permanent magnet (10) rotationally fixedly connected to the diverting element (8).

6. A magnetic coupling (1) as claimed in one of Claims 1 to 5, **characterised in that** a diamagnetic shielding element (7), which preferably peripherally adjoins the diverting element (8), is arranged on a front surface (15), directed towards the opposed permanent magnet (7), of the permanent magnet (10) rotationally fixedly connected to the diverting element (8).

7. A magnetic coupling (1) as claimed in one of Claims 1 to 6, **characterised in that** the two coupling portions (5, 6) are hermetically separated.

8. A magnetic coupling (1) as claimed in Claim 7, **characterised in that** for the hermetic separation of the two coupling portions at least one of the coupling portions (5, 6) is accommodated in a substantially non-magnetic and electrically non-conductive housing (14).

9. A pump with a drive and a pump rotor, wherein the pump rotor is connected to the drive via a magnetic coupling (1) as claimed in one of Claims 1 to 8.

10. Use of a pump as claimed in Claim 9 as a medical device, particularly as an implanted blood pump, preferably as a heart blood pump.

## Revendications

1. Embrayage magnétique (1) pour la transmission d'un couple le long d'un axe de rotation (4), avec deux parties d'embrayage (5, 6) tournant l'une par rapport à l'autre, une partie d'embrayage côté entrée (5) comprenant un aimant permanent côté entrée (10) et une partie d'embrayage côté sortie (6) comprenant un aimant permanent côté sortie (7), qui fait face à l'aimant permanent (10) le long de l'axe de rotation (4) et disposé à une certaine distance de celui-ci, une des parties d'embrayage (5 ; 6) comprenant au moins un élément de renvoi (8) au moins partiellement ferromagnétique, qui est relié de manière solidaire en rotation avec l'aiment permanent (10 ; 7) de la partie d'embrayage (5 ; 6), une partie de l'élément de renvoi (8) étant disposée radialement à l'extérieur de l'aimant permanent opposé (7 ; 10), **caractérisé en ce que** l'élément de renvoi (8) comprend au moins une séparation diamagnétique (9) qui divise l'élément de renvoi (8) en au moins deux portions ferromagnétiques (11, 12).

2. Embrayage magnétique (1) selon la revendication 1, **caractérisé en ce que** les deux aimants permanents (10, 7) sont chacun des aimants permanents à 2, 4 ou 6 pôles.

3. Embrayage magnétique (1) selon la revendication 1 ou 2, **caractérisé en ce que** l'élément de renvoi (8) s'étend sur un côté arrière (19), opposé à l'aimant permanent opposé (7 ; 10), de l'aimant permanent relié de manière solidaire en rotation (10 ; 7).

4. Embrayage magnétique (1) selon l'une des revendications 1 à 3, **caractérisé en ce que** l'élément de renvoi (8) comprend une enveloppe de forme cylindrique creuse (8') et de préférence est réalisé avec un fond intermédiaire (21) disposé globalement à mi-hauteur de l'enveloppe (8').

5. Embrayage magnétique (1) selon l'une des revendications 1 à 4, **caractérisé en ce que**, sur un côté arrière (19), opposé à l'aimant permanent opposé (7), de l'aimant permanent (10) relié de manière solidaire en rotation avec l'élément de renvoi, est disposé un élément de blindage diamagnétique (18, 25).

6. Embrayage magnétique (1) selon l'une des revendications 1 à 5, **caractérisé en ce que**, sur un côté avant (15), orienté vers l'aimant permanent opposé (7), de l'aimant permanent (10) relié de manière solidaire en rotation avec l'élément de renvoi (8), plus particulièrement dans une zone centrée autour de l'axe de rotation (4), un élément de blindage diamagnétique (17), qui se raccorde, de préférence sur la circonférence, à l'élément de renvoi (8).

7. Embrayage magnétique (1) selon l'une des revendications 1 à 6, **caractérisé en ce que** les deux parties de l'embrayage (5, 6) sont séparées de manière hermétique.

8. Embrayage magnétique (1) selon la revendication 7, **caractérisé en ce que**, pour la séparation hermétique des deux parties de l'embrayage, au moins une des parties de l'embrayage (5, 6) est logée dans un boîtier (14) globalement non magnétique et non électro-conducteur.

9. Pompe avec un dispositif d'entraînement et un rotor de pompe, le rotor de pompe étant relié avec le dispositif d'entraînement par l'intermédiaire d'un embrayage magnétique (1) selon l'une des revendications 1 à 8.

10. Utilisation d'une pompe selon la revendication 9 en tant qu'appareil médical, plus particulièrement en tant que pompe à sang implantée, de préférence en tant que pompe à sang cardiaque.
